# FASCICULE DE BREVET EUROPEEN

(11) **EP 1 578 442 B1**
(45) Date de publication et mention de la délivrance du brevet: **24.10.2012**
(21) Numéro de dépôt: 03815086.8
(22) Date de dépôt: 08.12.2003
(51) Int. Cl.: A61K 39/10, C12P 1/04

(54) **Procédé d'isolement d'endotoxines.**
Verfahren zur Isolierung von Endotoxinen.
Method for isolating endotoxins

(30) Priorité: 09.12.2002 FR 0215555
(43) Date de publication de la demande: 28.09.2005
(73) Titulaire: Université Paris-Sud 11, 91405 Orsay Cedex (FR)
(72) Inventeur: CAROFF, Martine, F-78140 Velizy (FR)
(74) Mandataire: Corizzi, Valérie
(86) Numéro de dépôt international: PCT/FR2003/003617
(87) Numéro de publication internationale: WO 2004/062690

(56) Documents cités:
- EP-A- 0 976 402
- CAROFF M G L ET AL: "SEVERAL USES FOR ISOBUTYRIC ACID-AMMONIUM HYDROXIDE SOLVENT IN ENDOTOXIN ANALYSIS" APPLIED AND ENVIRONMENTAL MICROBIOLOGY, vol. 56, no. 6, 1990, pages 1957-1959, XP008024672 ISSN: 0099-2240 cité dans la demande
- H. THERISOD ET AL: "Direct Microextraction and analysis of rough type Lipopolysaccharides by combined thin-layer Chromatography and MALDI spectrometry" ANALYTICAL CHEMISTRY, vol. 73, no. 16, 15 août 2001 (2001-08-15), pages 3804-3807, XP002262340 cité dans la demande
- RIDLEY BRENT L ET AL: "The type and yield of lipopolysaccharide from symbiotically deficient Rhizobium lipopolysaccharide mutants vary depending on the extraction method" GLYCOBIOLOGY, vol. 10, no. 10, octobre 2000 (2000-10), pages 1013-1023, XP008024739 ISSN: 0959-6658
- VALVERDE CLAUDIO ET AL: "Rapid preparation of affinity-purified lipopolysaccharide samples for electrophoretic analysis" BIOTECHNIQUES, vol. 22, no. 2, 1997, page 230, 232, 234, 236, XP001160910 ISSN: 0736-6205
- DI FABIO J I ET AL: "Characterization of the common antigenic lipopolysaccharide O-chains produced by Bordetella bronchiseptica and Bordetella parapertussis" FEMS (FEDERATION OF EUROPEAN MICROBIOLOGICAL SOCIETIES) MICROBIOLOGY, vol. 97, no. 3, 1992, pages 275-281, XP008024673 1992 ISSN: 0378-1097

## Description

L'invention a pour objet un nouveau procédé d'extraction d'endotoxines à partir de bactéries, en particulier de bactéries Gram-négatives, ainsi que l'utilisation de ce procédé pour la préparation de compositions comprenant des endotoxines ou leurs dérivés et destinées à l'usage humain ou animal (usage scientifique, médical...).

Les endotoxines sont de puissants stimulants du système immunitaire (induction d'Interleukines, TNF, NO...). Elles sont les constituants principaux des membranes externes des bactéries Gram-négatives. Les endotoxines sont des mélanges de lipopolysaccharides ou LPS.

Parmi les colonies bactériennes, on distingue principalement deux catégories : celles dites de type S, pour « smooth », se rapportant à l'aspect lisse de la colonie ; celles dites de type R, pour « rough », ce terme rappelant la morphologie rugueuse de la colonie. Il existe également une troisième catégorie dite SR pour « semi-rough ».

La structure des LPS constituants des endotoxines bactériennes est différente suivant qu'il s'agit de bactéries de type R ou S. Les LPS sont constitués d'un domaine lipidique dit lipide A et d'une chaîne polysaccharidique, dite de noyau, rattachée au lipide A.

Les LPS de type S comportent en outre une chaîne antigénique dite de type O. Les LPS de type R ne comportent pas cette chaîne antigénique de type O. Les LPS de type SR comportent une seule unité de la chaîne antigénique de type O.

La présente invention porte plus particulièrement sur l'isolement des endotoxines à partir d'une culture bactérienne de type R et éventuellement de type S-R. Toutefois, elle peut également être appliquée aux cultures bactériennes de type S.

Dans les procédés de l'art antérieur, les endotoxines bactériennes étaient isolées par extraction au moyen d'un mélange de solvants qui pouvait être un mélange de phénol et d'eau ou un mélange de chloroforme, d'éther de pétrole et de phénol. Ces méthodes présentent le grave inconvénient d'employer des solvants toxiques et donc d'entraîner des risques sanitaires pour les personnes amenées à les manipuler. Il existe également une méthode d'extraction à l'acide trichloracétique, toutefois cette méthode entraîne trop de contamination pour être jugée satisfaisante.

En outre, des résidus de solvants toxiques dans les extraits bactériens d'endotoxines les rendaient impropres à une administration humaine ou animale ainsi qu'à l'utilisation dans des expériences scientifiques. Enfin, l'élimination de ces résidus de solvants toxiques constituait une étape difficile et coûteuse du procédé de l'art antérieur.

Le document Caroff, M. and Karibian, D. « Several uses for Isobutyric Acid-Ammonium Hydroxide solvant in endotoxin analysis » Applied and Env. Microbiol. 56: 1957-1959 (1990), décrit l'utilisation du mélange de solvants acide isobutyrique NH₄OH molaire (5:3) pour déterminer la pureté d'endotoxines bactériennes par chromatographie ainsi que pour différencier des endotoxines bactériennes issues de différentes souches.

Ce document décrit aussi un procédé de purification d'endotoxines naturelles à partir d'endotoxines isolées suivant l'un des procédés traditionnels d'extraction au phénol dans une première étape. Dans la seconde étape de ce procédé, les endotoxines sont purifiées par extraction à l'aide du mélange de solvants acide isobutyrique / NH₄OH molaire (5 :3).

L'extrait est centrifugé et le surnageant récupéré.

Les solvants sont évaporés. L'endotoxine est récupérée par trituration à l'éthanol.

A la lecture de ce document, l'homme du métier comprend que l'extraction au phénol constitue la première étape incontournable de tout procédé d'isolement d'endotoxines bactériennes.

Le document Thérisod, H., Labas, V. and Caroff, M. « Direct microextraction and analysis of rough-type lipopolysaccharides by combined thin-layer chromatography and MALDI mass spectrometry ». Anal. Chem. 73: 3804-3807 (2001), décrit un procédé d'isolement d'endotoxines bactériennes, directement à partir de bactéries par chromatographie sur plaque de silice, l'éluant employé étant le mélange de solvants acide isobutyrique / NH₄OH (5:3).

Toutefois, la chromatographie sur plaque de silice et l'extraction aux solvants sont deux techniques séparatives bien distinctes et il n'existe pas de corrélation entre les résultats obtenus par ces deux techniques.

Aussi, c'est avec étonnement que la Demanderesse a découvert un nouveau procédé d'isolement d'endotoxines (ou lipopolysaccharides) bactériennes directement à partir de bactéries.

Le procédé selon l'invention se caractérise en ce que :
- le produit de départ est un ensemble de cellules bactériennes, ou éventuellement un surnageant de culture ;
- dans une première étape, les cellules bactériennes, ou le surnageant de culture, sont suspendus dans un mélange de solvants constitué de :
   ■ 10 à 90%, de préférence 30 à 70%, d'un solvant choisi parmi : les acides aliphatiques linéaires ou ramifiés comprenant de 3 à 6 atomes de carbone,
   ■ 90 à 10%, de préférence 70 à 30%, d'une solution aqueuse basique d'une amine aliphatique comprenant de 0 à 12 atomes de carbone,
   ■ et l'ensemble est agité, pour donner une solution dans laquelle des particules sont en suspension,
- dans une seconde étape, les particules en suspension sont séparées de la solution :
   ■ soit par filtration de la composition,
   ■ soit par centrifugation et récupération du surnageant,
   ■ soit par centrifugation suivie de la filtration du surnageant ;
   ■ dans une troisième étape, les solvants sont éliminés du surnageant ou du filtrat par tous moyens connus de l'homme du métier tels que notamment : dialyse, filtration / lavage, précipitation des LPS, par exemple par addition d'éthanol.

Le procédé selon la présente invention présente de nombreux avantages par rapport aux procédés de l'art antérieur. Il est rapide : l'extraction des endotoxines se fait en 12 à 24 heures ou moins, tandis que dans les procédés d'extraction au phénol, l'étape d'extraction elle-même durait au moins une semaine.

Cette méthode n'utilise pas de silice, elle est plus simple à mettre en oeuvre, notamment à l'échelle industrielle.

Cette méthode n'emploie pas de solvants toxiques, elle n'est donc pas dangereuse pour les manipulateurs qui la mettent en oeuvre.

Elle est très sélective.

Les rendements sont 2 à 10 fois plus élevés que ceux obtenus par les méthodes de l'art antérieur pour les bactéries déjà testées.

Les traitements enzymatiques habituels à la DNAse, RNAse et protéase, employés habituellement pour éliminer les contaminants en ADN, ARN, les peptides et les protéines résiduels, ne sont généralement pas nécessaires.

En outre, l'absence de solvants toxiques, la rapidité et les bons rendements du procédé de l'invention permettent d'envisager son extrapolation a l'échelle industrielle.

Enfin, de nombreux articles récents démontrent que certains LPS commercialisés actuellement, et obtenus par extraction dans un système phénol/eau, sont porteurs de contaminants qui les rendent inexploitables dans des expériences scientifiques. Les LPS obtenus par le procédé de l'invention présentent l'avantage de ne pas être porteurs de contaminants.

Parmi les acides aliphatiques utilisables dans la présente invention, on choisit préférentiellement un acide ramifié. On préfère les acides aliphatiques comprenant 4 atomes de carbone, tels que les acides butyrique et iso-butyrique. L'acide aliphatique préférentiellement utilisé dans la présente invention est l'acide iso-butyrique.

Parmi les amines aliphatiques utilisables dans la présente invention, on peut citer notamment : l'ammoniaque, la diisopropyléthylamine, la triéthylamine. On choisit préférentiellement l'ammoniaque ou la triéthylamine. Les amines aliphatiques sont employées en solution aqueuse d'une concentration allant de 5 à 20%, préférentiellement d'une concentration aux environs de 10%.

La composition résultant de la première étape est une composition hétérogène comportant d'une part des particules de débris cellulaires en suspension, d'autre part les endotoxines bactériennes en solution dans le mélange de solvants.

L'élimination des particules de débris cellulaires par filtration et/ou centrifugation permet d'isoler les endotoxines.

Si l'on souhaite purifier les endotoxines d'éventuels résidus contaminants, on peut extraire la composition issue de la troisième étape du procédé de l'invention (dialysat ou LPS précipité que l'on reprend dans l'eau) à l'aide de méthanol ou d'un mélange de chloroforme et de méthanol pour éliminer notamment des résidus phospholipidiques ou lipoprotéiques contaminants.

Suivant l'usage que l'on souhaite en faire, on peut conserver les endotoxines, issues du procédé de l'invention, en solution aqueuse ou les lyophiliser afin de les stocker sous forme de poudre, cette dernière solution ayant l'avantage de faciliter leur manipulation et leur dosage.

Les cellules bactériennes utilisées comme produit de départ sont préférentiellement des cellules Gram négatives. Toutefois, on peut également envisager d'utiliser le procédé selon l'invention pour purifier d'autres phosphoglycanes ou lipoglycanes isolés d'autres types bactériens, notamment à des fins vaccinales.

Des cellules préférentiellement utilisées dans le procédé de l'invention sont : *Bordetella pertussis*, *Escherichia Coli*, *Bordetella parapertussis*, *Salmonella*. *Shigella*, *Pseudomonas*, *Neisseria*, *Haemophilus*.

Les cellules bactériennes sont généralement obtenues par centrifugation d'un milieu de culture et récupération du culot bactérien. Toutefois, lors de cette opération de centrifugation il est possible de récupérer un surnageant qui peut être riche en lipopolysaccharides et peut constituer un produit de départ pour l'application du procédé de l'invention.

Les mélanges de solvants utilisés de façon préférentielle sont : l'acide isobutyrique et soit l'ammoniaque en solution aqueuse molaire soit la triéthylamine en solution aqueuse à 10% (pourcentage en volume).

De façon préférée, on utilise un mélange d'acide isobutyrique et d'ammoniaque en solution aqueuse molaire dans un rapport volumique 5/3 ou un mélange d'acide isobutyrique et de triéthylamine en solution aqueuse à 10% dans un rapport volumique 5/3.

De façon avantageuse, l'agitation correspondant à la première étape du procédé de l'invention dure de 5 à 15 minutes. Encore plus avantageusement de 10 à 15 minutes.

De préférence la filtration est faite sur un support d'une porosité allant de 0,1 à 2 µm.

Encore plus préférentiellement, de 0,2 à 0,45 µm.

Lorsque l'on centrifuge le mélange issu de la première étape, cette centrifugation se fait préférentiellement dans les conditions suivantes : 1500 à 3000g, pendant 10 à 20 minutes à température ambiante. Préférentiellement 2000g pendant environ 15 minutes.

Le procédé selon la présente invention permet d'obtenir les endotoxines de façon rapide, avec de très bons rendements et sensiblement exemptes de contaminants biologiques ou de solvants. Toutes ces propriétés permettent d'envisager des applications qui jusqu'alors pouvaient difficilement être mises en oeuvre.

Notamment la préparation de compositions destinées à l'administration humaine ou animale. En raison de la toxicité des solvants employés dans l'art antérieur et des difficultés de purification qu'ils entraînaient, de telles applications n'étaient pas envisageables. Grâce au procédé de l'invention, on peut à présent préparer des compositions, notamment des compositions thérapeutiques, qui peuvent être administrées à l'homme ou à l'animal (sous réserve de la non-toxicité des molécules elles-mêmes) par toutes voies : orale, injection, application sur la peau ou les muqueuses.

En particulier, il est possible de préparer, grâce à ce procédé, des compositions de vaccin comprenant des endotoxines, ou des dérivés d'endotoxines, à l'échelle industrielle.

Parmi les dérivés d'endotoxines, on peut citer notamment les endotoxines détoxifiées, les fragments d'endotoxines, les endotoxines conjuguées avec d'autres molecules, notamment avec des protéines.

L'utilisation d'endotoxines pour préparer des compositions de vaccins est citée notamment dans EP-976402.

L'invention a donc également pour objet un procédé pour la préparation d'une composition comprenant une ou plusieurs endotoxines ou dérivés d'endotoxines, tels que notamment des fragments d'endotoxine, des endotoxines détoxifiées, destinée à l'administration humaine ou animale, caractérisé en ce qu'il comporte une étape d'isolement des endotoxines telle que décrite ci-dessus. Dans un second temps, les endotoxines isolées sont incorporées dans un véhicule approprié, notamment un véhicule pharmaceutiquement acceptable. Elles peuvent aussi être conditionnées directement en vue d'une préparation extemporanée de la composition destinée à l'administration humaine ou animale.

Les endotoxines peuvent également être directement commercialisées pour un usage scientifique.

### Partie Expérimentale :

Des cellules sont mises en culture suivant une méthode déjà décrite par Di Fabio, J.L. ; Caroff, M., Karibian, D. ; Richards, J. C. and Perry, M. B. FEMS Microbiol. Lett. 1992, 76: 275-281.

L'homme du métier sait adapter les conditions de culture en fonction du type cellulaire concerné.

Un gramme de cellules lyophilisées (ou l'équivalent de culot bactérien humide) est suspendue dans 50 ml d'un mélange d'acide isobutyrique et de NH₄OH Molaire (5:3) et agitées doucement par agitation magnétique pendant 5 minutes. Le mélange est centrifugé à 2 000 g pendant 15 minutes à température ambiante. Le surnageant clarifié, contenant l'endotoxine, est filtré sur papier de verre (Whatman GF/D), dilué par trois volumes d'eau distillée et dialysé. Une seconde extraction du culot peut être effectuée si nécessaire. Le rétentat de la dialyse est filtré sur papier de verre dans les mêmes conditions que précédemment, puis lyophilisé, et il est extrait par un mélange de chloroforme et de méthanol (1:2) pour éliminer les contaminants éventuels de phospholipides. Le résidu de l'extraction est alors lyophilisé. Les deux étapes de filtration peuvent être réalisées sur verre fritté (16-40 µm) recouvert du papier de verre et un vide partiel peut être appliqué pour accélérer le procédé sous réserve de contrôle de la qualité du filtrat sur CCM.

Le rendement est de 2 à 10 % selon les bactéries.

L'extraction est réalisée en 24 heures. Cette durée peut être encore réduite à quelques heures par des procédés de dialyse par filtration, applicables à l'échelle industrielle.

Ces essais ont été effectués avec succès sur les bactéries du genre *Bordetella* (*B. pertussis*, agent de la coqueluche, et d'autres bactéries du même genre bactérien responsables d'infections respiratoires). Cette méthode a aussi été testée sur les cellules *d'Escherichia coli* K 12 et de *Neisseria meningitidis*.

Lorsque les bactéries comportent des polysaccharides capsulaires en grande quantité, il peut être avantageux de les éliminer par lavage des cellules avant de procéder à l'extraction.

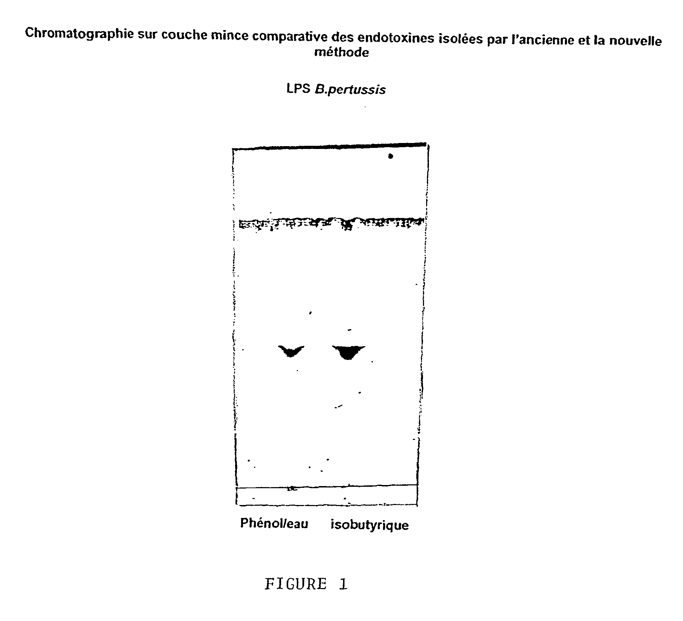

Une comparaison des endotoxines obtenues à partir de *Bordetella pertussis* par la présente méthode (notée isobutyrique) et la méthode au phénol-eau est illustrée par la figure 1, qui représente une chromatographie sur couche mince comparative des endotoxines isolées par ces deux méthodes. Une comparaison par gel SDS-PAGE, CCM et spectrométrie de masse a permis de vérifier l'efficacité de la méthode et l'intégrité des endotoxines.

## Revendications

1. Procédé d'isolement de lipopolysaccharides **caractérisé en ce que** :
- le produit de départ est un ensemble de cellules bactériennes ou un surnageant de culture ;
- dans une première étape, les cellules bactériennes, ou le surnageant de culture, sont suspendus dans un mélange de solvants constitué:
■ d'un solvant choisi parmi : les acides aliphatiques linéaires ou ramifiés comprenant de 3 à 6 atomes de carbone,
■ d'une solution aqueuse basique d'une amine aliphatique comprenant de 0 à 12 atomes de carbone, dans un rapport volumique allant de 1/9 à 9/1,
■ et l'ensemble est agité, pour donner une solution dans laquelle des particules sont en suspension,
- dans une seconde étape, les particules en suspension sont séparées de la solution
■ soit par filtration de la composition,
■ soit par centrifugation et récupération du surnageant,
■ soit par centrifugation suivie de la filtration du surnageant ;
- dans une troisième étape, les solvants sont éliminés du surnageant ou du filtrat.

2. Procédé selon la revendication 1, **caractérisé en ce que** les cellules bactériennes sont choisies parmi les bactéries Gram-négatives.

3. Procédé selon la revendication 2, **caractérisé en ce que** les cellules bactériennes sont choisies parmi les bactéries Gram-négatives de type R et S-R.

4. Procédé selon l'une quelconque des revendications 1 à 3, **caractérisé en ce que** les cellules sont choisies parmi : *Bordetella pertussis*, *Escherichia Coli*, *Bordetella parapertussis*, *Salmonella*, *Shigella*, *Pseudomonas*, *Neisseria*, *Haemophilus*.

5. Procédé selon l'une quelconque des revendications 1 à 4, **caractérisé en ce que** les solvants sont éliminés du surnageant ou du filtrat par une méthode choisie parmi : la dialyse, la filtration / lavage, la précipitation.

6. Procédé selon l'une quelconque des revendications 1 à 5, **caractérisé en ce que** l'on extrait la composition issue de la troisième étape du procédé à l'aide d'un mélange de chloroforme et de méthanol.

7. Procédé selon l'une quelconque des revendications 1 à 6, **caractérisé en ce que** le mélange de solvants est constitué:
■ d'un solvant choisi parmi : les acides aliphatiques linéaires ou ramifiés comprenant de 3 à 6 atomes de carbone,
■ d'une solution aqueuse basique d'une amine aliphatique comprenant de 0 à 12 atomes de carbone, dans un rapport volumique allant de 3/7 à 7/3.

8. Procédé selon l'une quelconque des revendications 1 à 7, **caractérisé en ce que** le mélange de solvants est choisi parmi: l'acide isobutyrique et la triéthylamine en solution aqueuse à 10% dans un rapport volumique 5/3.

9. Procédé selon l'une quelconque des revendications 1 à 7, **caractérisé en ce que** le mélange de solvants est un mélange d'acide isobutyrique et d'ammoniaque en solution aqueuse molaire dans un rapport volumique 5/3.

10. Procédé selon l'une quelconque des revendications 1 à 9, **caractérisé en ce que** l'agitation dans la première étape du procédé dure de 5 à 15 minutes.

11. Procédé selon l'une quelconque des revendications 1 à 10, **caractérisé en ce que** la filtration est faite sur un support d'une porosité allant de 0,1 à 2 µm.

12. Procédé selon l'une quelconque des revendications 1 à 11, **caractérisé en ce que** la centrifugation se fait dans les conditions suivantes : 1500 à 3000g, pendant 10 à 20 minutes à température ambiante.

13. Procédé pour la préparation d'une composition comprenant un ou plusieurs lipopolysaccharides ou dérivé de lipopolysaccharides, destinée à l'administration humaine ou animale, **caractérisé en ce qu'**il comporte un procédé d'isolement de lipopolysaccharides selon l'une quelconque des revendications 1 à 12.

14. Procédé selon la revendication 13, **caractérisé en ce que** la composition est une composition thérapeutique.

15. Procédé selon la revendication 14, **caractérisé en ce que** la composition est une composition de vaccin.

## Claims

1. Method of isolating lipopolysaccharides, **characterised in that**:
- the starting product is a set of bacterial cells or a culture supernatant;
- in a first step, the bacterial cells, or the culture supernatant, are suspended in a mixture of solvents made up of:
• a solvent selected from among: straight-chain or branched aliphatic acids having 3 to 6 carbon atoms,
• a basic aqueous solution of an aliphatic amine having 0 to 12 carbon atoms, in a ratio by volume ranging from 1/9 to 9/1,
• and the whole is agitated, to give a solution in which particles are suspended,
- in a second step, the suspended particles are separated from the solution
• either by filtration of the composition,
• or by centrifugation and recovery of the supernatant,
• or by centrifugation followed by filtration of the supernatant;
- in a third step, the solvents are eliminated from the supernatant or the filtrate.

2. Method according to claim 1, **characterised in that** the bacterial cells are selected from among the Gram-negative bacteria.

3. Method according to claim 2, **characterised in that** the bacterial cells are selected from among the Gram-negative bacteria of type R and S-R.

4. Method according to any one of claims 1 to 3, **characterised in that** the cells are selected from among: *Bordetella pertussis*, *Escherichia Coli*, *Bordetella parapertussis*, *Salmonella*, *Shigella*, *Pseudomonas*, *Neisseria*, *Haemophilus*.

5. Method according to any one of claims 1 to 4, **characterised in that** the solvents are eliminated from the supernatant or the filtrate by a method selected from among: dialysis, filtration/lavage, precipitation.

6. Method according to any one of claims 1 to 5, **characterised in that** the composition resulting from the third step of the method is extracted using a mixture of chloroform and methanol.

7. Method according to any one of claims 1 to 6, **characterised in that** the mixture of solvents is made up of:
• a solvent selected from among: straight-chain or branched aliphatic acids having 3 to 6 carbon atoms,
• a basic aqueous solution of an aliphatic amine having 0 to 12 carbon atoms, in a ratio by volume ranging from 3/7 to 7/3.

8. Method according to any one of claims 1 to 7, **characterised in that** the mixture of solvents is selected from among: isobutyric acid and triethylamine in 10% aqueous solution in a ratio by volume of 5/3.

9. Method according to any one of claims 1 to 7, **characterised in that** the mixture of solvents is a mixture of isobutyric acid and ammonia in molar aqueous solution in a ratio by volume of 5/3.

10. Method according to any one of claims 1 to 9, **characterised in that** the agitation in the first step of the method lasts 5 to 15 minutes.

11. Method according to any one of claims 1 to 10, **characterised in that** the filtration is carried out on a support with a porosity ranging from 0.1 to 2 µm.

12. Method according to any one of claims 1 to 11, **characterised in that** the centrifugation is carried out under the following conditions: 1500 to 3000 g, for 10 to 20 minutes, at ambient temperature.

13. Method for preparing a composition comprising one or more lipopolysaccharides or lipopolysaccharide derivatives, intended for administration to humans or animals, **characterised in that** it comprises a method of isolating lipopolysaccharides according to any one of claims 1 to 12.

14. Method according to claim 13, **characterised in that** the composition is a therapeutic composition.

15. Method according to claim 14, **characterised in that** the composition is a vaccine composition.

## Patentansprüche

1. Verfahren zum Isolieren von Lipopolysacchariden, **dadurch gekennzeichnet, dass**
- das Ausgangsprodukt eine Gesamtheit von bakteriellen Zellen oder der Überstand einer Kultur ist;
- in einem ersten Schritt die bakteriellen Zellen bzw. der Kulturüberstand in einer Mischung von Lösungsmitteln suspendiert werden, bestehend aus:
• einem Lösungsmittel, das aus geradkettigen oder verzweigten aliphatischen Säuren mit 3 bis 6 Kohlenstoffatomen ausgewählt ist,
• einer basischen wässrigen Lösung eines aliphatischen Amins mit 0 bis 12 Kohlenstoffatomen in einem Volumenverhältnis von 1/9 bis 9/1,
• wobei die Gesamtheit gerührt wird, um eine Lösung zu erhalten, in der die Partikel in Suspension vorliegen,
- in einem zweiten Schritt die in Suspension vorliegenden Partikel
• entweder durch Filtrieren der Zusammensetzung
• oder durch Zentrifugieren und Wiedergewinnen des Überstands
• oder durch Zentrifugieren, gefolgt von Filtrieren des Überstands von der Lösung abgetrennt werden,
- in einem dritten Schritt die Lösungsmittel aus dem Überstand bzw. dem Filtrat entfernt werden.

2. Verfahren nach Anspruch 1, **dadurch gekennzeichnet, dass** die bakteriellen Zellen unter den gramnegativen Bakterien ausgewählt werden.

3. Verfahren nach Anspruch 2, **dadurch gekennzeichnet, dass** die bakteriellen Zellen unter den gramnegativen Bakterien vom Typ R und S-R ausgewählt werden.

4. Verfahren nach einem der Ansprüche 1 bis 3, **dadurch gekennzeichnet, dass** die Zellen aus *Bordetella pertussis*, *Escherichia Coli*, *Bordetella parapertussis*, *Salmonella*, *Shigella*, *Pseudomonas*, *Neisseria*, *Haemophilus* ausgewählt werden.

5. Verfahren nach einem der Ansprüche 1 bis 4, **dadurch gekennzeichnet, dass** die Lösungsmittel aus dem Überstand oder dem Filtrat mittels eines Verfahrens entfernt werden, das aus Dialyse, Filtration / Waschen, Fällung ausgewählt ist.

6. Verfahren nach einem der Ansprüche 1 bis 5, **dadurch gekennzeichnet, dass** die aus dem dritten Schritt des Verfahrens hervorgegangene Zusammensetzung mithilfe einer Mischung aus Chloroform und Methanol extrahiert wird.

7. Verfahren nach einem der Ansprüche 1 bis 6, **dadurch gekennzeichnet, dass** die Lösungsmittelmischung aus
• einem Lösungsmittel, das aus geradkettigen oder verzweigten aliphatischen Säuren mit 3 bis 6 Kohlenstoffatomen ausgewählt ist,
• einer basischen wässrigen Lösung eines aliphatischen Amins mit 0 bis 12 Kohlenstoffatomen in einem Volumenverhältnis von 3/7 bis 7/3 besteht.

8. Verfahren nach einem der Ansprüche 1 bis 7, **dadurch gekennzeichnet, dass** die Lösungsmittelmischung aus Isobuttersäure und Triethylamin in 10%-iger wässriger Lösung in einem Volumenverhältnis von 5/3 ausgewählt wird.

9. Verfahren nach einem der Ansprüche 1 bis 7, **dadurch gekennzeichnet, dass** die Lösungsmittelmischung eine Mischung aus Isobuttersäure und Ammoniak in molarer wässriger Lösung in einem Volumenverhältnis von 5/3 ist.

10. Verfahren nach einem der Ansprüche 1 bis 9, **dadurch gekennzeichnet, dass** das Rühren im ersten Schritt des Verfahrens 5 bis 15 Minuten dauert.

11. Verfahren nach einem der Ansprüche 1 bis 10, **dadurch gekennzeichnet, dass** das Filtrieren auf einem Träger mit einer Porosität von 0,1 bis 2 µm durchgeführt wird.

12. Verfahren nach einem der Ansprüche 1 bis 11, **dadurch gekennzeichnet, dass** das Zentrifugieren unter den folgenden Bedingungen durchgeführt wird: 1500 bis 3000 g, während 10 bis 20 Minuten bei Raumtemperatur.

13. Verfahren zur Bereitung einer Zusammensetzung, welche ein oder mehrere Lipopolysaccharide oder Lipopolysaccharid-Derivate aufweist und zur Verabreichung an Mensch oder Tier bestimmt ist, **dadurch gekennzeichnet, dass** es ein Verfahren zum Isolieren von Lipopolysacchariden nach einem der Ansprüche 1 bis 12 umfasst.

14. Verfahren nach Anspruch 13, **dadurch gekennzeichnet, dass** es sich bei der Zusammensetzung um eine therapeutische Zusammensetzung handelt.

15. Verfahren nach Anspruch 14, **dadurch gekennzeichnet, dass** es sich bei der Zusammensetzung um eine Impfstoffzusammensetzung handelt.
